# EUROPÄISCHE PATENTANMELDUNG

(11) **EP 4 645 325 A1**
(43) Veröffentlichungstag der Anmeldung: **05.11.2025**
(21) Anmeldenummer: 24174234.5
(22) Anmeldetag: 04.05.2024
(51) Int. Cl.: G16H 10/60, G06N 3/08, G06N 20/00

(54) **VERFAHREN ZUR ERZEUGUNG VON MEDIZINISCHEN BERICHTEN**

(30) Priorität: 02.05.2024 EP 24173968
(71) Anmelder: myScribe GmbH, 68167 Mannheim (DE)
(72) Erfinder: STOLL, Lars, 68526 Ladenburg (DE); STOLL, Ira, 68526 Ladenburg (DE)
(74) Vertreter: Best, Bastian

(57) **Zusammenfassung**

Offenbart ist ein computer-implementiertes Verfahren zum Erzeugen von medizinischen Berichten, insbesondere Arztbriefen. Das Verfahren kann einen Schritt eines Empfanges von medizinischen Informationen über einen Patienten umfassen. Die empfangenen medizinischen Informationen können in natürlichsprachlichen Stichpunkten formuliert sein. Das Verfahren kann einen Schritt eines Erzeugens von natürlichsprachlichem Fließtext, welcher die empfangenen medizinischen Informationen widerspiegelt, für einen medizinischen Bericht durch ein trainiertes generatives Sprachmodell, umfassen.

## Beschreibung

### TECHNISCHES GEBIET

Die vorliegende Erfindung betrifft im Allgemeinen das Feld der generativen Sprachmodelle und im speziellen ein Verfahren zum Erzeugen von medizinischen Berichten unter Verwendung eines trainierten generativen Sprachmodells.

### HINTERGRUND

Medizinische Berichte sind essentiell im Gesundheitswesen. Sie verursachen jedoch oftmals einen erheblichen Mehraufwand für das medizinische Personal. Dieser Mehraufwand kann dazu führen, dass die Versorgungsqualität der Patienten sinkt und/oder die Arbeitsbelastung der medizinischen Fachangestellten steigt. Die erhöhte Arbeitsbelastung kann beispielsweise dazu führen, dass medizinische Berichte geringer Qualität erstellt werden, wodurch die nachfolgende Behandlung der Patienten auf einer nicht vollständigen Informationsbasis erfolgt. Die geringe Qualität der medizinischen Berichte lässt sich beispielsweise daran festmachen, dass einige Aspekte nur mangelhaft beschrieben oder teilweise in Gänze fehlen.

Somit ist es ein Ziel der vorliegenden Erfindung ein Verfahren zum Erzeugen von medizinischen Berichten bereitzustellen, welches die zuvor genannten Nachteile des Stands der Technik zumindest teilweise überwindet.

### ZUSAMMENFASSUNG DER ERFINDUNG

Dies wird durch den in den unabhängigen Ansprüchen definierten Gegenstand gelöst. Vorteilhafte Modifikationen von Ausführungsformen der vorliegenden Offenbarung sind in den abhängigen Ansprüchen sowie in der Beschreibung und den Figuren definiert.

In einem ersten Aspekt betrifft die Erfindung ein computer-implementiertes Verfahren zum Erzeugen von medizinischen Berichten, insbesondere Arztbriefen. Das Verfahren kann einen Schritt eines Empfangens von medizinischen Informationen über einen Patienten umfassen. Die empfangenen medizinischen Informationen können in natürlichsprachlichen Stichpunkten formuliert sein. Das Verfahren kann einen Schritt eines Erzeugens von natürlichsprachlichem Fließtext, welcher die empfangenen medizinischen Informationen widerspiegelt, für einen medizinischen Bericht durch ein trainiertes generatives Sprachmodell umfassen.

Dadurch wird die automatische Erzeugung von qualitativ hochwertigen medizinischen Berichten mittels des generativen Sprachmodells ermöglicht. Dass die Erzeugung ausgehend von natürlichsprachlichen Stichpunkten durchgeführt werden kann, verringert den damit verbundenen Mehraufwand für das medizinische Personal, da dieses die Stichpunkte lediglich in natürlicher Sprache bereitstellen müssen. Diese können beispielsweise über ein Textfeld einer APP oder auch über eine Spracherkennungsschnittstelle (z.B. eines Smartphones, eines Tabletts, einer Smartwatch, eines Diktiergeräts etc.) bereitgestellt werden. Somit wird ein System bereitgestellt das dem wachsenden Bedarf an effizienter, standardisierter und zeitnaher medizinischer Dokumentation in Gesundheitseinrichtungen Rechnung trägt, indem es den Verwaltungsaufwand für medizinisches Fachpersonal erheblich reduziert.

Gemäß einem weiteren Aspekt kann das generative Sprachmodell so konfiguriert sein, dass der erzeugte natürlichsprachliche Fließtext alle und nur die empfangenen medizinischen Informationen widerspiegelt.

Dadurch wird verhindert, dass das Modell medizinische Informationen erzeugt, welche nicht in der Eingabe enthalten sind. Ebenfalls wird verhindert, dass das Modell medizinische Informationen bei der Erzeugung weglässt. Dadurch wird sichergestellt, dass alle relevanten medizinischen Informationen Teil des erzeugten Berichts sind, aber auch keine falschen Informationen enthalten sind. Dadurch kann eine nachfolgende Behandlung des Patienten auf Basis einer vollständigen Informationslage gewährleistet werden. Dadurch kann eine Zulassung des Verfahrens als Medizinprodukt erfolgen.

Gemäß einem weiteren Aspekt kann das generative Sprachmodell so konfiguriert sein, dass der natürlichsprachliche Fließtext die empfangenen Informationen deterministisch widerspiegelt.

Da in den meisten Fällen der medizinische Bericht nach der Erzeugung von einem medizinischen Fachangestellten kontrolliert wird, ist es hilfreich, wenn das Modell die empfangenen Informationen deterministisch widerspiegelt. Würde das Modell nicht deterministisch arbeiten, könnte sich der erzeugte Fließtext bei jeder Erzeugung unterscheiden, wodurch das medizinische Personal immer den kompletten Bericht kontrollieren müsste. Ist das Modell jedoch deterministisch, müssen alle Teile des Berichts nur einmal kontrolliert werden (d.h. bei Hinzufügen einer neuen medizinischen Information muss auch nur der dazugehörige und entsprechend neu erzeugte Teil des medizinischen Berichts kontrolliert werden).

Gemäß einem weiteren Aspekt kann der Schritt des Erzeugens von natürlichsprachlichem Text umfassen: Bereitstellen aller in den empfangenen medizinischen Informationen enthaltenen Informationen gesammelt und/oder in einem Schritt an das generative Sprachmodell, um den natürlichsprachlichen Fließtext zu erzeugen. Durch diese Technik, welche auch als "One-Shot-Prompting" bezeichnet werden kann, kann der als Ergebnis erzeugte natürlichsprachliche Fließtext besonders effizient erzeugt werden, da das generative Sprachmodell sämtliche Informationen in einem einzigen Eingabeprompt erhält und somit nur einen einzigen Eingabeprompt verarbeiten muss, um den natürlichsprachlichen Fließtext zu erzeugen.

Alternativ zu dem obigen Aspekt kann der Schritt des Erzeugens von natürlichsprachlichem Text umfassen: Bereitstellen der in den empfangenen medizinischen Informationen enthaltenen Informationen jeweils einzeln an das generative Sprachmodell, um jeweils ein Zwischenergebnis zu erzeugen; Kombinieren der Zwischenergebnisse zu einem Gesamt-Zwischenergebnis; und Bereitstellen des Gesamt-Zwischenergebnisses an das generative Sprachmodell, um den natürlichsprachlichen Fließtext zu erzeugen. Die Zwischenergebnisse können in natürlichsprachlichem Fließtext erzeugt werden. Das Gesamt-Zwischenergebnis kann durch ein Aneinanderreihen der Zwischenergebnisse erzeugt werden. Durch diese Technik, welche auch als "Chain of Summary" bzw. "Chain of Density Prompting" bezeichnet werden kann, kann der als Ergebnis erzeugte natürlichsprachliche Fließtext in manchen Anwendungsszenarien detailreicher sein, ohne zu dicht bzw. schwer verständlich zu sein.

Es kann ferner vorgesehen sein, dass das Verfahren bzw. ein das Verfahren ausführendes Programm beide wie oben beschriebene Arten des Erzeugens von natürlichsprachlichem Text unterstützt. Die für eine individuelle empfangene Eingabe mit empfangenen medizinischen Informationen zu wählende Art des Erzeugens kann beispielsweise durch einen Benutzer konfigurierbar sein.

Gemäß einem weiteren Aspekt kann der Schritt des Empfanges von medizinischen Informationen einen Schritt eines Empfanges von zumindest einer Benutzereingabe in einer grafischen Benutzeroberfläche, insbesondere durch medizinisches Fachpersonal während einer Visite eines Patienten, umfassen. Zusätzlich oder alternativ kann der Schritt des Empfangens von medizinischen Informationen einen Schritt eines Empfangens von zumindest einem elektronischen Datensatz aus einem Krankenhausinformationssystem umfassen. Der zumindest eine elektronische Datensatz kann "Health Level 7"-konform und/oder "Fast Healthcare Interoperability Resources"-konform sein. Zusätzlich oder alternativ kann das Verfahren einen Schritt eines Erzeugens des medizinischen Berichts als elektronische Datei, welche den erzeugten natürlichsprachlichen Fließtext enthält, umfassen.

Dadurch wird ein voll-digitaler Workflow zur Erzeugung der medizinischen Berichte bereitgestellt, welcher sich unkompliziert in existierende Krankenhausinformationssysteme einbinden lässt. Durch den voll-digitalen Workflow kann die Qualität der medizinischen Berichte erhöht werden (z.B. keine Kopierfehler) und gleichzeitig der mit der Erzeugung verbundene Aufwand reduziert werden.

Gemäß einem weiteren Aspekt können die empfangenen medizinischen Informationen zumindest eine medizinische Information umfassen, welche einer der folgenden Kategorien angehört: Geschlecht, Leitsymptom, Diagnosen, Behandlungen, Medikamente, Befunde und Verlaufsdokumentation.

Mit Hilfe dieser medizinischen Informationen lässt sich eine vollständige medizinische Dokumentation abbilden.

Gemäß einem weiteren Aspekt kann das Verfahren einen Schritt eines Kategorisierens der empfangenen medizinischen Informationen in die Kategorien umfassen. Das Kategorisieren kann mittels eines Klassifikators, insbesondere eines statistischen Klassifikators, erfolgen.

Gemäß einem weiteren Aspekt kann das Verfahren einen Schritt eines Erzeugens einer Eingabekodierung für das generative Sprachmodell basierend auf den empfangenen medizinischen Informationen umfassen. Die Eingabekodierung kann die medizinischen Informationen so kodieren, dass eine maximale Eingabelänge des generativen Sprachmodells berücksichtigt wird.

Somit wird eine kompakte Kodierung der medizinischen Informationen bereitgestellt, wodurch beispielsweise eine maximale Eingabelänge des generativen Sprachmodells berücksichtigt werden kann. Außerdem kann durch die kompakte Kodierung, die zu verarbeitende Datenmenge reduziert werden.

Gemäß einem weiteren Aspekt kann die Eingabekodierung so erzeugt werden, dass jede medizinische Information in der Eingabekodierung einem vorgegebenen Kategorie-Kürzel, welches die jeweilige Kategorie anzeigt, zugeordnet ist.

Dadurch wird eine besonders kompakte Kodierung bereitgestellt, welche jedoch durch die Kategorie-Kürzel mit keinem Informationsverlust einhergeht.

Gemäß einem weiteren Aspekt kann die Eingabekodierung so erzeugt werden, dass die Eingabekodierung für zumindest eine der Kategorien mehrere medizinische Informationen derselben Kategorie enthält. Das Kategorie-Kürzel kann dabei nur einmal in der Eingabekodierung enthalten sein. Die Eingabekodierung kann so erzeugt werden, dass die mehreren medizinischen Informationen derselben Kategorie durch ein vorgegebenes Trenn-Kürzel getrennt sind.

Dadurch wird eine besonders kompakte Kodierung bereitgestellt. Dadurch, dass eine Kategorie mehrere medizinische Informationen enthalten kann, ermöglicht die Eingabekodierung die Abbildung eines vollständigen und umfangreichen Behandlungsprozesses. Das Trenn-Kürzel ermöglicht es dabei, dass die einzelnen medizinischen Informationen identifizierbar bleiben und keine Vermischung der medizinischen Informationen stattfindet. Dies könnte ansonsten zu einem medizinischen Bericht führen, welcher inkorrekte medizinische Informationen enthält.

Gemäß einem weiteren Aspekt kann die Eingabekodierung so erzeugt werden, dass zumindest einer medizinischen Information in der Eingabekodierung ein Zeitstempel zugeordnet ist, insbesondere nach der medizinischen Information und durch ein vorgegebenes Trenn-Kürzel getrennt.

Somit wird eine besonders kompakten Kodierung bereitgestellt, welche einen zeitlichen Verlauf exakt darstellen kann.

Gemäß einem weiteren Aspekt kann das Kategorie-Kürzel und/oder das Trenn-Kürzel genau ein Zeichen enthalten.

Dadurch wird eine besonders Kompakte Kodierung bereitgestellt, welche trotzdem die eindeutige Zuordnung der medizinischen Informationen zu Kategorien als auch die eindeutige Differenzierbarkeit mehrerer medizinischen Informationen derselben Kategorie ermöglicht.

In einem zweiten Aspekt betrifft die Erfindung ein generatives Sprachmodell zum Erzeugen von medizinischen Berichten, insbesondere Arztbriefen. Das generative Sprachmodell kann zum Erzeugen von natürlichsprachlichem Fließtext für einen medizinischen Bericht konfiguriert sein. Der erzeugte natürlichsprachliche Fließtext kann medizinische Informationen über einen Patienten widerspiegeln. Die medizinischen Informationen können in natürlichsprachlichen Stichpunkten formuliert sein. Das generative Sprachmodell kann zur Verwendung im Verfahren nach irgendeinem der vorhergehenden Aspekte konfiguriert sein.

In einem dritten Aspekt betrifft die Erfindung eine Eingabekodierungs-Datenstruktur für das generative Sprachmodel gemäß dem zweiten Aspekt. Die Eingabekodierung kann nach irgendeinem der vorherigen Aspekte konfiguriert sein.

In einem vierten Aspekt betrifft die Erfindung ein Verfahren zum Trainieren des generativen Sprachmodells gemäß dem zweiten Aspekt. Das Verfahren kann einen Schritt eines Bereitstellens von Trainingsdatensätzen umfassen. Ein Trainingsdatensatz kann eine Eingabekodierungs-Datenstruktur gemäß dem dritten Aspekt umfassen. Ein Trainingsdatensatz kann einen natürlichsprachlichen Fließtext für einen medizinischen Bericht umfassen. Das Verfahren kann einen Schritt eines Trainierens des generativen Sprachmodells durch überwachtes Lernen mit den Trainingsdatensätzen umfassen.

In einem fünfen Aspekt betrifft die Erfindung eine Datenverarbeitungsvorrichtung umfassend Mittel zum Durchführen der Verfahren nach irgendeinem der erwähnten Aspekte.

In einem sechsten Aspekt betrifft die Erfindung ein Computerprogramm oder ein computerlesbares Medium, auf dem ein Computerprogramm gespeichert ist, wobei das Computerprogramm Anweisungen umfasst, die, wenn das Computerprogramm von einem Computer ausgeführt wird, den Computer veranlassen, das Verfahren nach irgendeinem der erwähnten Aspekte auszuführen.

### KURZBESCHREIBUNG DER FIGUREN

Die Erfindung kann anhand der folgenden Figuren besser verstanden werden:
- Fig. 1:: Ein Flussdiagramm eines computer-implementierten Verfahrens zum Erzeugen von medizinischen Berichten gemäß einer beispielhaften Ausführungsform der vorliegenden Erfindung.
- Fig. 2:: Ein Flussdiagramm eines Verfahrens zum Trainieren eines generativen Sprachmodells gemäß einer beispielhaften Ausführungsform der vorliegenden Erfindung.
- Fig. 3:: Eine Architektur eines generativen Sprachmodells zum Erzeugen von medizinischen Berichten gemäß einer beispielhaften Ausführungsform der vorliegenden Erfindung.
- Fig. 4a:: Eine Eingabekodierungs-Datenstruktur für ein generative Sprachmodel gemäß einer beispielhaften Ausführungsform der vorliegenden Erfindung.

- Fig. 4b:: Ein medizinischer Bericht gemäß einer beispielhaften Ausführungsform der vorliegenden Erfindung.
- Fig. 5:: Eine Datenverarbeitungsvorrichtung gemäß einer beispielhaften Ausführungsform der vorliegenden Erfindung.

### DETAILLIERTE BESCHREIBUNG

Im Folgenden werden repräsentative Ausführungsformen, die in den beigefügten Zeichnungen dargestellt sind, erläutert. Es sollte verstanden werden, dass die abgebildeten Ausführungsformen und die folgenden Beschreibungen sich auf Beispiele beziehen, die nicht dazu gedacht sind, die Ausführungsformen auf eine bevorzugte Ausführungsform zu beschränken.

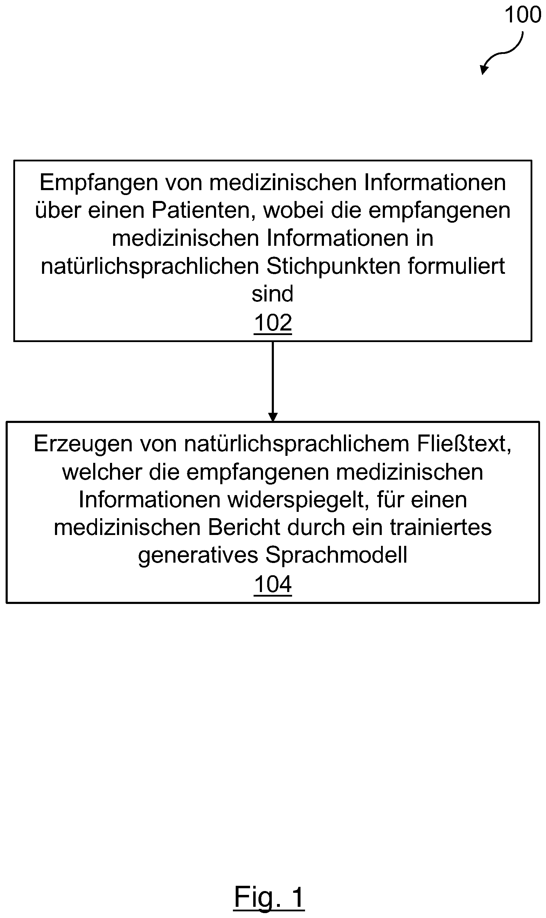

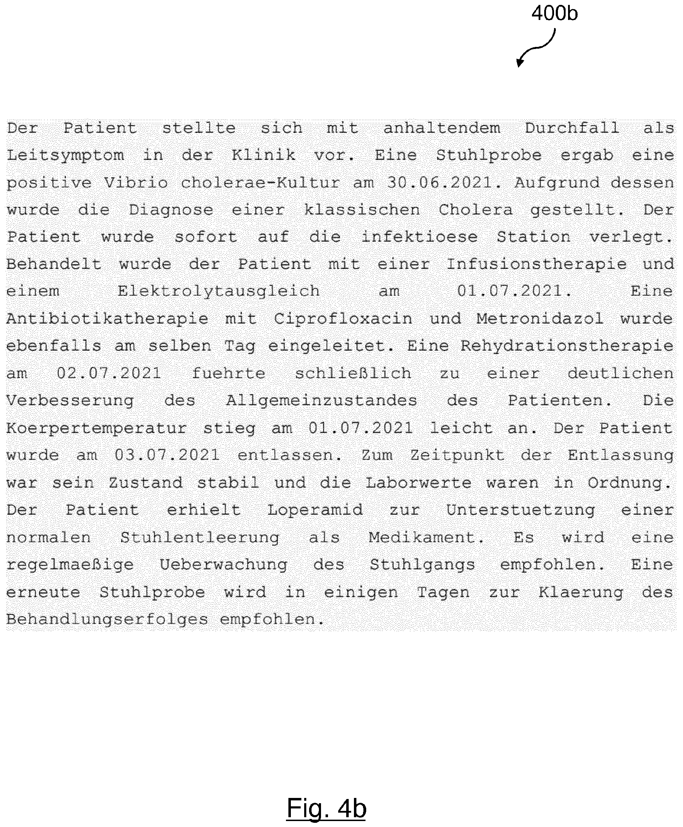

Fig. 1 zeigt ein Flussdiagramm eines computer-implementierten Verfahrens 100 zum Erzeugen von medizinischen Berichten gemäß einer beispielhaften Ausführungsform der vorliegenden Erfindung. Bei den medizinischen Berichten kann es sich um Arztbriefe handeln. Bei dem medizinischen Bericht kann es sich um einen wie in Fig. 4b gezeigten medizinischen Bericht handeln.

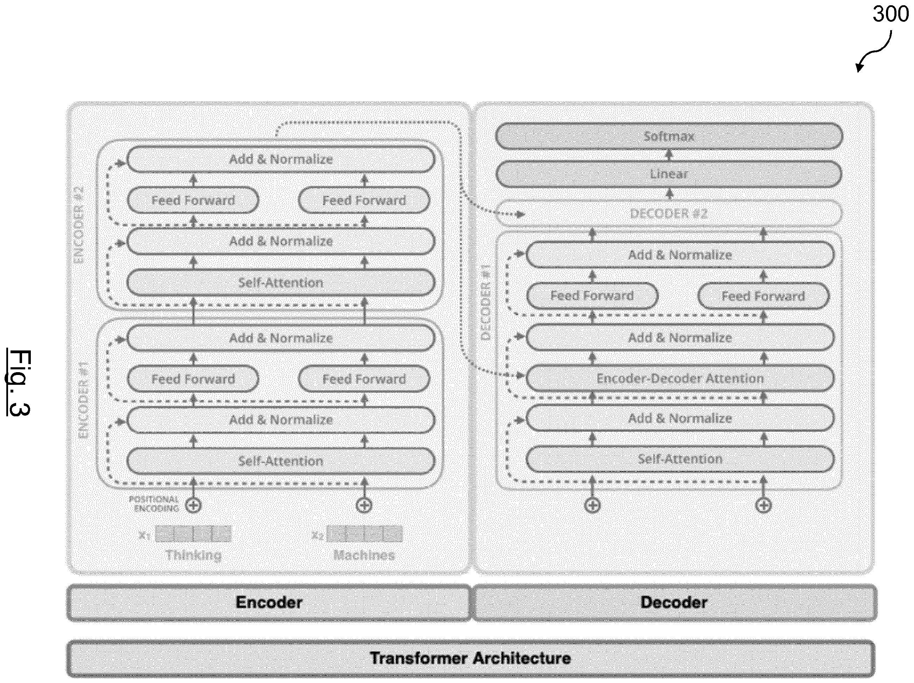

Das Verfahren 100 kann einen Schritt eines Empfangens 102 von medizinischen Informationen über einen Patienten umfassen. Die empfangenen medizinischen Informationen können in natürlichsprachlichen Stichpunkten formuliert sein. Das Verfahren 100 kann einen Schritt eines Erzeugens 104 von natürlichsprachlichem Fließtext, welcher die empfangenen medizinischen Informationen widerspiegelt, für einen medizinischen Bericht durch ein trainiertes generatives Sprachmodell umfassen. Dabei kann es sich um ein, wie in Fig. 3 gezeigtes generatives Sprachmodell handeln.

Das generative Sprachmodell kann so konfiguriert sein, dass der erzeugte natürlichsprachliche Fließtext alle und nur die empfangenen medizinischen Informationen widerspiegelt. Das generative Sprachmodell kann so konfiguriert sein, dass der natürlichsprachliche Fließtext die empfangenen Informationen deterministisch widerspiegelt.

Der Schritt des Empfangens 102 von medizinischen Informationen kann einen Schritt eines Empfangens von zumindest einer Benutzereingabe in einer grafischen Benutzeroberfläche, insbesondere durch medizinisches Fachpersonal während einer Visite eines Patienten, umfassen. Zusätzlich oder alternativ kann der Schritt des Empfangens 102 von medizinischen Informationen einen Schritt eines Empfangens von zumindest einem elektronischen Datensatz aus einem Krankenhausinformationssystem umfassen. Der zumindest eine elektronische Datensatz kann "Health Level 7"-konform und/oder "Fast Healthcare Interoperability Resources"-konform sein. Zusätzlich oder alternativ kann das Verfahren 100 einen Schritt eines Erzeugens des medizinischen Berichts als elektronische Datei, welche den erzeugten natürlichsprachlichen Fließtext enthält, umfassen.

Medizinische Informationen über Patienten können in einer Datenbank gespeichert sein. Bei der Datenbank kann es sich um eine Krankenhaus-interne Datenbank handeln. Das Empfangen des zumindest einen elektronischen Datensatzes aus dem Krankenhausinformationssystem kann umfassen, dass eine Anfrage an einen Krankenhausserver gesendet wird. Der Krankenhausserver kann Teil des Krankenhausinformationssystem sein und/oder Zugriff auf die Krankenhaus-interne Datenbank haben. Bei der Anfrage kann es sich um eine HTTPS-Anfrage handeln. Dies ermöglicht eine unkomplizierte Implementierung bzw. Integration der vorliegenden Erfindung in ein existierendes Krankenhausinformationssystem, da andere Schnittstellen (z.B. TCP-IP) zwischen dem Krankenhausserver bzw. der Krankenhaus-internen Datenbank und anderen Komponenten des Krankenhausinformationssystems (z.B. Backend und/oder Frontend) unverändert gelassen werden können.

Die empfangenen medizinischen Informationen können zumindest eine medizinische Information umfassen, welche einer der folgenden Kategorien angehört: Geschlecht, Leitsymptom, Diagnosen, Behandlungen, Medikamente, Befunde und Verlaufsdokumentation.

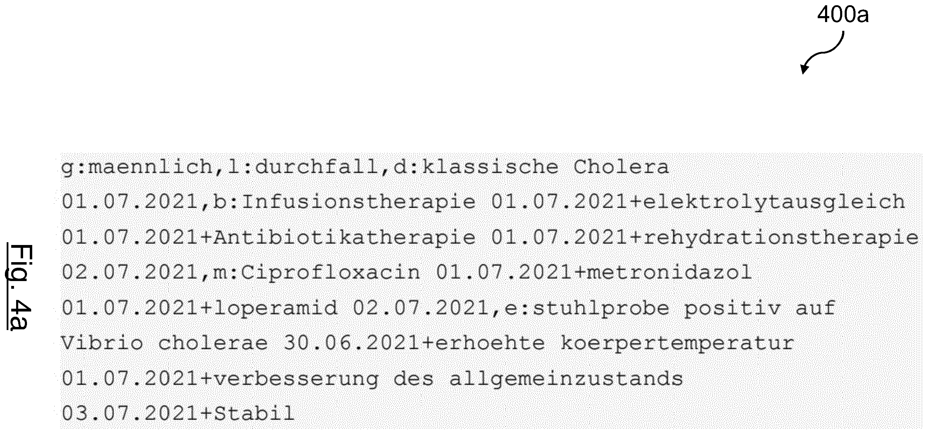

Das Verfahren 100 kann einen Schritt eines Erzeugens einer Eingabekodierung für das generative Sprachmodell basierend auf den empfangenen medizinischen Informationen umfassen. Die Eingabekodierung kann die medizinischen Informationen so kodieren, dass eine maximale Eingabelänge des generativen Sprachmodells berücksichtigt wird. Die Eingabekodierung kann so erzeugt werden, dass jede medizinische Information in der Eingabekodierung einem vorgegebenen Kategorie-Kürzel, welches die jeweilige Kategorie anzeigt, zugeordnet ist. Die Eingabekodierung kann so erzeugt werden, dass die Eingabekodierung für zumindest eine der Kategorien mehrere medizinische Informationen derselben Kategorie enthält. Das Kategorie-Kürzel kann dabei nur einmal in der Eingabekodierung enthalten sein. Die Eingabekodierung kann so erzeugt werden, dass die mehreren medizinischen Informationen derselben Kategorie durch ein vorgegebenes Trenn-Kürzel getrennt sind. Die Eingabekodierung kann so erzeugt werden, dass zumindest einer medizinischen Information in der Eingabekodierung ein Zeitstempel zugeordnet ist, insbesondere nach der medizinischen Information und durch ein vorgegebenes Trenn-Kürzel getrennt. Das Kategorie-Kürzel und/oder das Trenn-Kürzel kann genau ein Zeichen enthalten. Bei der Eingabekodierung kann es sich um eine wie in Fig. 4a gezeigte Eingabekodierung handeln.

Unabhängig davon, ob die oben beschriebene Eingabekodierung verwendet wird oder die medizinischen Informationen anderweitig kategorisiert werden, kann durch die Verwendung des Verfahrens 100 die Dauer einer Patientenaufnahme erheblich verkürzt werden. Praktische Versuche haben Folgendes ergeben: Dauer der Anamnese von 10 Minuten auf 2 Minuten, Dauer der körperlichen Untersuchung von 10 auf 5 Minuten. Geht man von einer Dauer von 5 Minuten für die Erfassung der Medikamente aus, kann somit eine Zeitersparnis von 13 Minuten (d.h. 12 statt 25 Minuten) erzielt werden.

Durch die Verwendung des Verfahrens 100 kann die Dauer einer Visite wie folgt verkürzt werden: Dauer der Vorarbeit von 5 Minuten auf 0 Minuten, Dauer der Nacharbeit von 5 auf 0 Minuten. Geht man von einer Dauer von 5 Minuten für die Visitendokumentation aus, kann somit eine Zeitersparnis von 10 Minuten (d.h. 5 statt 15 Minuten) erzielt werden.

Durch die Verwendung des Verfahrens 100 kann die Dauer einer Patientenentlassung wie folgt verkürzt werden: Dauer der Arztbriefschreibung von 30-60 Minuten auf 5 Minuten, wobei davon die Dauer des Kopierens alter Befunde von 15 auf 0 Minuten und die Dauer der Epikrisen Schreibung von 15-45 auf 5 Minuten beträgt. Geht man von einer Dauer von 5 Minuten für die Therapieempfehlung aus, kann somit eine Zeitersparnis von 25-55 Minuten (d.h. 5 statt 35-65 Minuten) erzielt werden. Hierbei wird von einer durchschnittlichen Liegedauer von 4 Tagen ausgegangen.

Durch die Verwendung des Verfahrens 100 kann außerdem eine Zeitersparnis von 65-72% erzielt werden (d.h. 42 statt 120-150 Minuten).

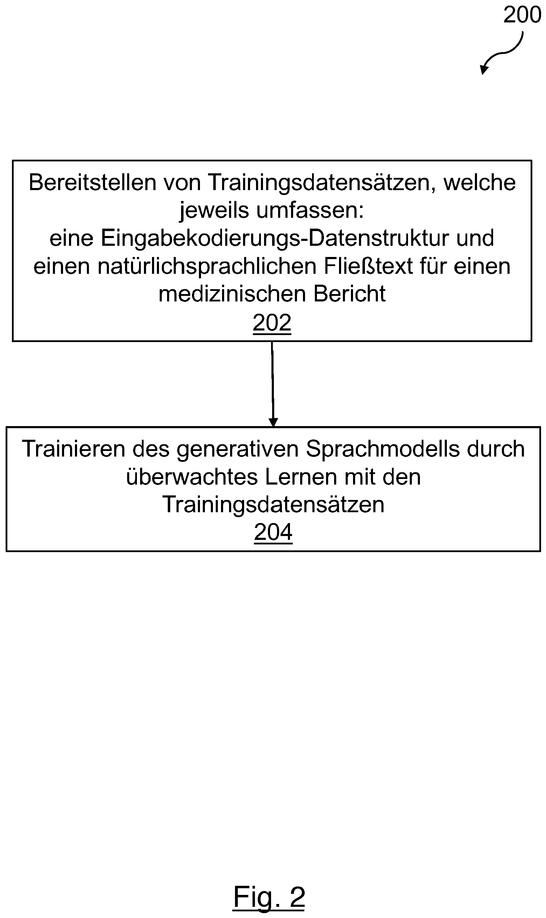

Fig. 2 zeigt ein Flussdiagramm eines Verfahrens 200 zum Trainieren eines generativen Sprachmodells gemäß einer beispielhaften Ausführungsform der vorliegenden Erfindung. Dabei kann es sich um ein wie in Fig. 3 gezeigtes generatives Sprachmodell handeln.

Das Verfahren 200 kann einen Schritt eines Bereitstellens 202 von Trainingsdatensätzen umfassen. Ein Trainingsdatensatz kann eine Eingabekodierungs-Datenstruktur gemäß Aspekten der vorliegenden Erfindung umfassen. Es kann sich beispielsweise um eine Eingabekodierungs-Datenstruktur wie in Fig. 4a gezeigt oder um anderweitig kategorisierte Eingabeinformationen handeln. Ein Trainingsdatensatz kann einen natürlichsprachlichen Fließtext für einen medizinischen Bericht umfassen. Bei dem medizinischen Bericht kann es sich um einen medizinischen Bericht wie in Fig. 4b gezeigt handeln. Das Verfahren 200 kann einen Schritt eines Trainierens 204 des generativen Sprachmodells durch überwachtes Lernen mit den Trainingsdatensätzen umfassen.

Beim Trainieren kann zunächst ein erster Satz von Trainingsdaten erzeugt werden. Bei dem ersten Satz von Trainingsdaten kann es sich um einen künstlich erzeugten Trainingsdatensatz handeln. Unter Verwendung des ersten Satzes kann das generative Sprachmodell vortrainiert werden. Dieses vortrainierte generative Sprachmodell kann dann bereitgestellt werden (z.B. in ein Krankenhausinformationssystem integriert werden) und mittels eines zweiten Satzes von Trainingsdaten erneut trainiert bzw. fine-getuned werden. Bei dem zweiten Satz von Trainingsdaten kann es sich um einen Satz von echten Trainingsdaten handeln. In diesem Kontext bezeichnet "echt", dass es ich dabei um medizinische Informationen von Patienten des entsprechenden Krankenhausinformationssystems handelt. Somit kann ein besser auf die individuellen Patientenarten (z.B. Patienten für entsprechende Fachkliniken) abgestimmtes Sprachmodell bereitgestellt werden.

Vor dem Trainieren des Modells kann eine Daten-Vorverarbeitung durchgeführt werden, bei der die Trainingsdaten in einen Trainings-, einen Validierungs- und einen Testdatensatz unterteilt werden.

Beim Trainieren des Modells kann eine Verlustfunktion verwendet werden. Die Verlustfunktion kann eine Verlustfunktion sein, die für sequenzielle Generierungsaufgaben geeignet ist. Beispielsweise kann die Verlustfunktion eine Kreuzentropie-Verlustfunktion sein. Die Verlustfunktion kann die Inkonsistenz zwischen der vorhergesagten Ausgabe des Modells (z.B. den natürlichsprachlichen Fließtext eines erzeugten medizinischen Berichts) und einer tatsächlichen Zielausgabe (z.B. den dazugehörigen, tatsächlichen natürlichsprachlichen Fließtext des tatsächlichen medizinischen Berichts) messen. Basierend auf dieser Messung kann das Modell angeleitet werden, den Fehler zwischen der vorhergesagten Ausgabe und der Zielausgabe zu minimieren, um dadurch seine Vorhersagegenauigkeit zu verbessern.

Beim Trainieren des Modells kann ein Optimierer verwendet werden. Der Optimierer kann ein für große Modelle (d.h. hinsichtlich der Größe bzw. Tiefe des Modells) und große Datenmengen (z.B. hinsichtlich der Anzahl an Daten sowie der Dateigröße einer einzelnen Trainingsdatei) spezialisierter Optimierer sein. Der Optimierer kann alternativ oder zusätzlich ein für Transformer-Modelle geeigneter (d.h. hinsichtlich der Effizienz des Optimierers) Optimierer sein. Der Optimierer kann beispielsweise ein Adafactor-Optimierer sein.

Beim Trainieren des Modells kann eine Generierungsfunktion verwendet werden. Ein Parametersatz für die Generierungsfunktion kann umfassen: Eingabe-Token-IDs (z.B. 512 Ids bei einer maximalen Eingabelänge des generativen Sprachmodells 300 von 512), eine Anzahl der höchsten Wahrscheinlichkeiten aus denen ausgewählt wird (z.B. 50), einen Schwellenwert zur Beschränkung der Wahrscheinlichkeit der ausgewählten Tokens (z.B. 0.95), einen Faktor zur Beeinflussung der Wahrscheinlichkeitsverteilung (z.B. 0.3, wobei ein Wert < 1 zu einer deterministischen Ausgabe führt, einen Early-Stopping-Indikator (z.B. True, damit die Generierung frühzeitig gestoppt wird, wenn die End-of-Sequence-Bedingung erfüllt ist), eine Anzahl an Strahlen/Beams für einen Beam-Search-Algorithmus.

Beim Trainieren des Modells kann ein Satz von Hyperparametern verwendet werden. Der Satz von Hyperparametern kann beispielsweise eine Batch-Größe und eine Lernrate umfassen. Der Satz von Hyperparametern kann einen Untersatz an Parametern umfassen, welche zur Parametrisierung des verwendeten Optimierers verwendet werden. Der Untersatz an Parametern kann eine Lernrate, einen Lernratenabfallrate, einen ersten Epsilon Wert, einen zweiten Epsilon Wert, einen Clipping-Schwellenwert, einen adaptiven Lernratenindikator, einen Gewichteabfallwert, einen Clipping-Norm Wert, einen Clipping-Wert, einen globalen Clipping-Norm Wert, einen EMA ("Expontential Moving Average")-Indikator, oder eine beliebige Kombination daraus. Die Lernrate kann 0.001 betragen. Die Lernratenabfallrate kann -0.8 betragen. Der erste Epsilon Wert kann einen Abstand angeben, um einen Denominator nicht null werden zu lassen. Der erste Epsilon Wert kann beispielsweise einen Wert von 1e-30 annehmen. Der zweite Epsilon Wert kann einen Abstand angeben, um eine zu kleinwerdende Lernrate zu verhindern. Der zweite Epsilon Wert kann beispielsweise einen Wert von 1e-3 annehmen. Der Clipping-Schwellenwert kann beispielsweise einen Wert von 1 annehmen. Der adaptive Lernratenindikator kein einen Boolean-Wert annehmen, wobei "True" angibt, dass eine adaptive Lernrate (d.h., die Lernrate wird basierend auf der aktuellen Trainingsiteration angepasst) verwendet wird und "False" angibt, dass keine adaptive Lernrate verwendet wird. Der Gewichteabfallwert kann einen Float-Wert annehmen. Vorzugweise ist der Gewichteabfallwert null, sodass kein Gewichte Abfall stattfindet. Der Clipping-Norm-Wert kann einen Float-Wert annehmen. Der Clipping-Norm-Wert kann einen Wert anzeigen, welchen die Norm eines Gradienten eines jeden Gewichts nicht übersteigen darf (d.h., auf welchen die Norm eines Gewichts geclipped wird). Der Clipping-Wert kann einen Float-Wert annehmen. Der Clipping-Wert kann einen Wert anzeigen, welchen den Wert eines Gradienten eines jeden Gewichts nicht übersteigen darf (d.h., auf welchen der Wert eines Gewichts geclipped wird). Der globale Clipping-Norm-Wert kann einen Wert anzeigen, welchen die globale Norm aller Gradienten aller Gewichte nicht übersteigen dürfen. Der EMA-Indikator kann einen Boolean-Wert annehmen, wobei "True" anzeigt, dass ein EMA verwendet wird und wobei "False" anzeigt, dass kein EMA verwendet wird. Bei EMA wird ein exponentieller, bewegender Mittelwert basierend auf den Gewichten des Modells ermittelt, welcher periodisch die tatsächlichen Gewichtswerte überschreibt. Der EMA-Momentumwert kann einen Float-Wert, vorzugsweise 0,.99 annehmen. Der EMA-Momentumwert kann nur verwendet werden, falls der EMA-Indikator "True" ist. Die EMA-Überschreibungsfrequenz kann einen eine positive, ganze Zahl einschließlich der Null sein. Die EMA-Überschreibungsfrequenz kann nur verwendet werden, falls der EMA-Indikator "True" ist. Falls die EMA-Überschreibungsfrequenz null ist, werden keine Modelvariablen (z.B. Gewichte) während dem Training überschrieben. Falls die EMA-Überschreibungsfrequenz i ist, wobei *i*>*0,* dann werden jede *i-te* Iteration die Modellvariablen (z.B. Gewichte) mit dem bewegenden Mittelwert überschrieben. Der Verlustskalierungsfaktor kann einen Float-Wert annehmen. Falls der Wert gleich null ist, wird kein Verlustskalierungsfaktor angewendet. Andernfalls wird der Verlustskalierungsfaktor mit dem Verlust multipliziert, bevor die Gradienten berechnet werden. Die Gradienten-Akkumulierungsschrittweite kann einen Integer-Wert einschließlich der Null annehmen. Bei Null wird keine Akkumulierungsschrittweite verwendet (d.h., die Model und/oder Optimierer Variablen werden bei jeder Iteration aktualisiert). Andernfalls werden die Variablen (z.B. Gewichte) nur jede i-te Iteration aktualisiert.

Fig. 3 zeigt eine Architektur eines generativen Sprachmodells 300 zum Erzeugen von medizinischen Berichten gemäß einer beispielhaften Ausführungsform der vorliegenden Erfindung. Bei dem medizinischen Bericht kann es sich um einen wie in Fig. 4b gezeigten medizinischen Bericht handeln. Bei den medizinischen Berichten kann es sich um Arztbriefe handeln. Das generative Sprachmodell 300 kann zum Erzeugen von natürlichsprachlichem Fließtext für einen medizinischen Bericht konfiguriert sein. Der erzeugte natürlichsprachliche Fließtext kann medizinische Informationen über einen Patienten widerspiegeln. Die medizinischen Informationen können in natürlichsprachlichen Stichpunkten formuliert sein. Das generative Sprachmodell 300 kann zur Verwendung im Verfahren 100 konfiguriert sein. Das generative Sprachmodell 300 kann mittels des Verfahrens 200 trainiert sein.

Bei dem generativen Sprachmodell 300 kann es sich um ein auf Transformatoren basierendes generatives Sprachmodell handeln. Beispielsweise kann es sich dabei um ein Text-zu-Text Transformer Modell wie einem T5-Modell oder einem Mistral-Modell handeln.

Das generative Sprachmodell 300 kann ein zur Abarbeitung von sequenziellen Aufgaben konfiguriertes Modell sein.

Die in Fig. 3 gezeigte beispielhafte Architektur entspricht der Architektur eines auf dem T5 Modell basierenden generativen Sprachmodell 300. Das generative Sprachmodell 300 umfasst einen Encoder- und einen Decoder-Teil. Wie gezeigt kann sowohl der Encoder- als auch der Decoder-Teil auf dem Transformer-Design basieren. Einer oder beide Teile können mehrere Schichten von Multi- Head-Self-Attention Mechanismen und Feed-Forward-Netzwerken umfassen. Das generative Sprachmodell 300 kann eine spezielle Einbettung für Tokens und Positionen umfassen. Die spezielle Einbettung kann am Anfang des Modells hinzugefügt sein.

Der Encoder-Teil kann konfiguriert sein die Eingabe (z.B. strukturierte Stichpunkte) zu empfangen und so zu verarbeiten, dass eine kontextreiche Darstellung der Eingabe erstellt wird. Diese kontextreiche Darstellung dient wiederum als Eingabe für den Decoder-Teil, welcher basierend auf der kontextreichen Darstellung einen Ausgabetext (z.B. einen oder mehrere Abschnitte des medizinischen Berichts) erzeugt. Dabei kann der Decoder-Teil beim Erzeugen eines weiteren Ausgabetextes (z.B. einem oder mehreren nachfolgenden Abschnitten des medizinischen Berichts) den zuvor erzeugten Ausgabetext als Eingabe verwenden, wodurch dem Decoder zusätzliche Kontextinformation bereitgestellt werden kann.

Dabei können die strukturierten Stichpunkte, welche beispielsweise in Form einer Eingabekodierung (wie zu Fig. 4a erklärt) vorliegen können, tokenisiert werden. Bei der Tokenisierung wird die Eingabekodierung in eine numerische Repräsentation umgewandelt. Aus der numerischen Repräsentation kann dann die kontextreiche Darstellung erstellt werden.

Zur Erzeugung des Programmiercodes für das generative Sprachmodell 300 kann beispielsweise das "Transformers"-Software Paket verwendet werden, welches eine Sammlung von vortrainierten Modellen und Funktionalitäten für Natural Language Processing bietet. Zusätzlich kann eine Bibliothek zur Text Tokenisierung (z.B. "Sentencepiece") verwendet werden.

Fig. 4a zeigt eine Eingabekodierungs-Datenstruktur 400a für ein generative Sprachmodell 300 gemäß einer beispielhaften Ausführungsform der vorliegenden Erfindung.

Wie gezeigt, kann die Eingabekodierung 400a strukturierte Stichpunkte beinhalten, welche verschiedene medizinische Informationen repräsentieren. In anderen Worten, die Eingabekodierung 400a für das generative Sprachmodell 300 kann basierend auf empfangenen medizinischen Informationen erzeugt werden. Die Eingabekodering 400a ermöglicht eine effiziente und kompakte Repräsentation der medizinischen Information wodurch einerseits kein Informationsverlust und andererseits eine effiziente Verarbeitung (z.B. hinsichtlich der benötigten Verarbeitungsressourcen) erzielt wird.

Die Kompaktheit der Eingabekodierung 400a kann in einer möglichen Ausführungsform durch die Verwendung entsprechender Eingabe- bzw. Kategorie-Kürzel erzielt werden, wodurch der benötigte Datenumfang reduziert werden kann (z.B. "g" für Geschlecht, "l" für Leitsymptom, "d" für Diagnose, "b" für Behandlung und/oder "e" für Epikrise). In anderen Worten, die Eingabekodierung 400a kann so erzeugt werden, dass jede medizinische Information in der Eingabekodierung 400a einem vorgegebenen Kategorie-Kürzel, welches die jeweilige Kategorie anzeigt, zugeordnet ist. Zum Beispiel kann das Kategorie-Kürzel "g" das Geschlecht des Patienten angeben. Die Verwendung der Kategorie-Kürzel kann insbesondere dann von Bedeutung sein, wenn das verwendete, generative Sprachmodell 300 eine maximalige Eingabelänge (z.B. 512 Token) aufweist. In anderen Worten. Die Eingabekodierung 400a kann die medizinischen Informationen so kodieren, dass eine maximale Eingabelänge des generativen Sprachmodells 300 berücksichtigt wird. Dadurch können überflüssige Wörter/Zeichen vermieden werden und stattdessen eine Formatierung bereitgestellt werden, welche einen geringen Speicherbedarf aufweist. Der geringe Speicherbedarf ermöglicht es ebenso, dass eine große Menge an medizinischen Informationen in einem kompakten, maschinenlesbaren Format (d.h., der Eingabekodierung) übermittelt werden können, wodurch das generative Sprachmodell 300 detaillierte und nuancierte medizinische Berichte generieren kann.

Die Eingabekodierung 400a kann so erzeugt werden, dass die Eingabekodierung 400a für zumindest eine der Kategorien mehrere medizinische Informationen derselben Kategorie enthält. Zum Beispiel enthält die gezeigte Eingabekodierung 400a mehrere medizinische Informationen der Kategorie "b" (d.h. Behandlung).

Die Eingabekodierung 400a kann so erzeugt werden, dass die mehreren medizinischen Informationen derselben Kategorie durch ein vorgegebenes Trenn-Kürzel getrennt sind. Zum Beispiel sind die mehreren medizinischen Informationen der Kategorie "b" durch das vergebene Trenn-Kürzel "+" voneinander getrennt. Hierdurch wird eine klare Differenzierung zwischen den mehreren medizinischen Informationen ermöglicht. Durch diese Differenzierbarkeit können mehrere Behandlungen, Medikamente oder Verlaufsdokumentation (Beobachtungen) in einer einzelnen Eingabekodierung 400a (z.B. einem Eingabestring) spezifiziert werden, während die medizinischen Informationen klar voneinander getrennt bleiben.

Die Eingabekodierung 400a kann so erzeugt werden, dass zumindest einer medizinischen Information in der Eingabekodierung 400a ein Zeitstempel zugeordnet ist, insbesondere nach der medizinischen Information und durch ein vorgegebenes Trenn-Kürzel getrennt. Das Kategorie-Kürzel und/oder das Trenn-Kürzel kann genau ein Zeichen enthalten. Zum Beispiel kann eine Behandlung "Infusionstherapie" einen Zeitstempel zugeordnet bekommen (z.B. 01.07.2021), welcher den Zeitpunkt der Durchführung der entsprechenden Behandlung anzeigt. Obwohl in dem gezeigten Beispiel der Zeitstempel nach der medizinischen Information angezeigt wird, kann dies auch vor der medizinischen Information angezeigt werden. Zusätzlich kann ein "Leerzeichen" zwischen der medizinischen Information und dem zugehörigen Zeitstempel benutzt werden. Die Zeitstempel (z.B. Datum) ermöglichen die Darstellung eines zeitlichen Ablauf bzw. Verlauf der Symptome, Diagnosen und Behandlungen.

Das Kategorie-Kürzel kann dabei nur einmal in der Eingabekodierung 400a enthalten sein. Zum Beispiel enthält die gezeigte Eingabekodierung 400a nur einmal die Kategorie-Kürzel "g", "l", "d", "b" und "e".

Die Eingabekodierung 400a kann auf Basis von natürlichsprachlichen Stichpunkten erzeugt werden. Die natürlichsprachlichen Stichpunkte können beispielsweise von medizinischem Personal bereitgestellt werden (z.B. über ein Textfeld einer APP und/oder eine Spracherkennungsschnittstelle). Die gezeigte Eingabekodering 400a kann beispielsweise aus einem rohen Input (d.h., den natürlichsprachlichen Stichpunkten) erzeugt werden, welcher neben anderen Informationen (z.B. dem Geschlecht), umfasst: "Fieber, Sturz aus dem Bett, 2 Erythrozytenkonzentrate, Erbrochen".

Fig. 4b zeigt einen medizinischen Bericht 400b gemäß einer beispielhaften Ausführungsform der vorliegenden Erfindung. Bei dem medizinischen Bericht 400b kann es sich um einen Arztbrief handeln.

Der medizinische Bericht 400b kann erzeugten natürlichsprachliche Fließtext umfassen, welcher alle empfangenen medizinischen Informationen aber auch nur die empfangenen medizinischen Informationen widerspiegelt. Der natürlichsprachliche Fließtext kann die empfangenen Informationen widerspiegeln. Der medizinische Bericht kann als elektronische Datei, welche den erzeugten natürlichsprachlichen Fließtext enthält, erzeugt werden.

Wie man sehen kann, enthält der medizinische Bericht 400b alle medizinischen Informationen der Eingabekodierung 400a. Außerdem enthält der medizinische Bericht 400b keine medizinischen Informationen, welche nicht auch in der Eingabekodierung 400a enthalten sind. Zudem kann der medizinische Bericht 400b bzw. der entsprechende natürlichsprachliche Fließtext die medizinischen Informationen der Eingabekodierung 400a deterministisch widerspiegeln. In anderen Worten, basierend auf der Eingabekodering 400a wird immer derselbe natürlichsprachliche Fließtext für den medizinischen Bericht 400b erzeugt.

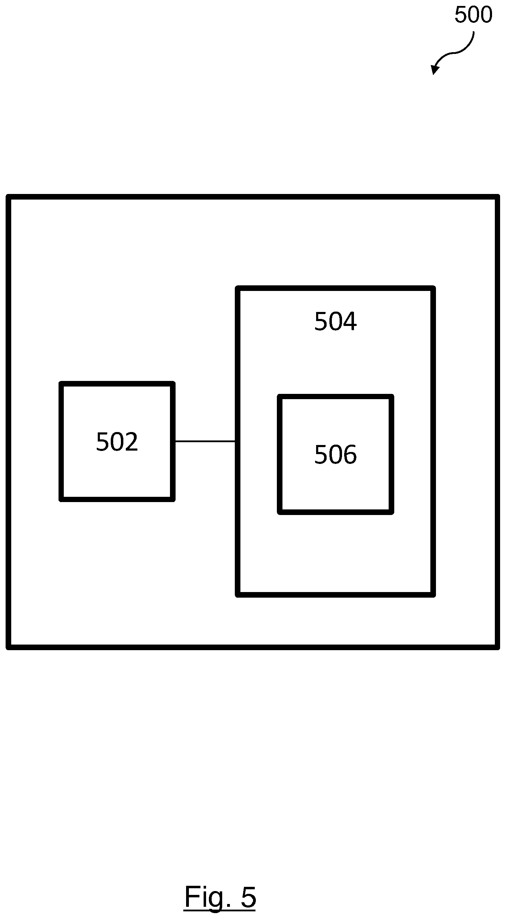

Fig. 5 zeigt eine Datenverarbeitungsvorrichtung 500 gemäß einer beispielhaften Ausführungsform der vorliegenden Erfindung.

Die Datenverarbeitungsvorrichtung kann Mittel zum Durchführen der Verfahren umfassen (z.B. die Verfahren 100 und/oder 200). Bei den Mitten kann es sich um einen Prozessor 502 und einen Speicher 504 handeln. Der Prozessor 502 und der Speicher 504 können wirkverbunden sein. Im Speicher 504 kann ein Computerprogramm gespeichert ist, wobei das Computerprogramm Anweisungen umfasst, die, wenn das Computerprogramm von einem Computer ausgeführt wird, den Computer veranlassen, das Verfahren nach irgendeinem der erwähnten Aspekte (z.B. das Verfahren 100 und/oder 200) auszuführen.

Der hier verwendete Begriff "und/oder" schließt alle Kombinationen von einem oder mehreren der aufgeführten Aspekte ein und kann mit "/" abgekürzt werden.

Obwohl einige Aspekte im Zusammenhang mit einem Gerät beschrieben wurden, ist es klar, dass diese Aspekte auch eine Beschreibung des entsprechenden Verfahrens darstellen, wobei ein Block oder eine Vorrichtung einem Verfahrensschritt oder einem Merkmal eines Verfahrensschritts entspricht. Analog dazu stellen Aspekte, die im Zusammenhang mit einem Verfahrensschritt beschrieben werden, auch eine Beschreibung eines entsprechenden Blocks oder Elements oder Merkmals eines entsprechenden Geräts dar.

Ausführungsformen der vorliegenden Offenbarung können auf einem Computersystem implementiert werden. Bei dem Computersystem kann es sich um ein lokales Computergerät (z. B. Personalcomputer, Laptop, Tablet-Computer oder Mobiltelefon) mit einem oder mehreren Prozessoren und einem oder mehreren Speichergeräten handeln oder um ein verteiltes Computersystem (z. B. ein Cloud-Computersystem mit einem oder mehreren Prozessoren und einem oder mehreren Speichergeräten, die an verschiedenen Orten verteilt sind, z. B. an einem lokalen Client und/oder einer oder mehreren entfernten Serverfarmen und/oder Datenzentren). Das Computersystem kann jede beliebige Schaltung oder Kombination von Schaltungen umfassen. In einer Ausführungsform kann das Computersystem einen oder mehrere Prozessoren umfassen, die von beliebigem Typ sein können. Der hier verwendete Begriff "Prozessor" kann jede Art von Rechenschaltung bezeichnen, z. B. einen Mikroprozessor, einen Mikrocontroller, einen CISC-Mikroprozessor (Complex Instruction Set Computing), einen RISC-Mikroprozessor (Reduced Instruction Set Computing), einen VLIW-Mikroprozessor (Very Long Instruction Word), einen Grafikprozessor, einen digitalen Signalprozessor (DSP), einen Mehrkernprozessor, ein FPGA (Field Programmable Gate Array) oder jede andere Art von Prozessor oder Verarbeitungsschaltung. Andere Arten von Schaltkreisen, die im Computersystem enthalten sein können, können ein kundenspezifischer Schaltkreis, ein anwendungsspezifischer integrierter Schaltkreis (ASIC) oder ähnliches sein, wie z. B. ein oder mehrere Schaltkreise (z. B. ein Kommunikationsschaltkreis) zur Verwendung in drahtlosen Geräten wie Mobiltelefonen, Tablet-Computern, Laptop-Computern, Zwei-Wege-Funkgeräten und ähnlichen elektronischen Systemen. Das Computersystem kann eine oder mehrere Speichervorrichtungen enthalten, die ein oder mehrere für die jeweilige Anwendung geeignete Speicherelemente umfassen können, wie z. B. einen Hauptspeicher in Form eines Direktzugriffsspeichers (RAM), eine oder mehrere Festplatten und/oder ein oder mehrere Laufwerke, die mit Wechselmedien wie Compact Discs (CD), Flash-Speicherkarten, digitalen Videodisks (DVD) und dergleichen umgehen. Das Computersystem kann auch ein Anzeigegerät, einen oder mehrere Lautsprecher und eine Tastatur und/oder ein Steuergerät enthalten, das eine Maus, einen Trackball, einen Touchscreen, ein Spracherkennungsgerät oder jedes andere Gerät umfassen kann, das es einem Systembenutzer ermöglicht, Informationen in das Computersystem einzugeben und Informationen von ihm zu empfangen.

Einige oder alle Verfahrensschritte können von einem Hardware-Gerät (oder unter Verwendung eines solchen) ausgeführt werden, wie z. B. einem Prozessor, einem Mikroprozessor, einem programmierbaren Computer oder einer elektronischen Schaltung. In einigen Ausführungsformen können einige oder mehrere der wichtigsten Verfahrensschritte von einem solchen Gerät ausgeführt werden.

Abhängig von bestimmten Implementierungsanforderungen können Ausführungsformen der vorliegenden Offenbarung in Hardware oder in Software implementiert werden. Die Implementierung kann unter Verwendung eines nicht-übertragbaren Speichermediums wie eines digitalen Speichermediums, beispielsweise einer Diskette, einer DVD, einer Blu-Ray, einer CD, eines ROM, eines PROM, eines EPROM, eines EEPROM oder eines FLASH-Speichers erfolgen, auf dem elektronisch lesbare Steuersignale gespeichert sind, die mit einem programmierbaren Computersystem zusammenarbeiten (oder zusammenarbeiten können), so dass das jeweilige Verfahren durchgeführt wird. Daher kann das digitale Speichermedium computerlesbar sein.

Einige Ausführungsformen gemäß der vorliegenden Offenbarung umfassen einen Datenträger mit elektronisch lesbaren Steuersignalen, die mit einem programmierbaren Computersystem zusammenarbeiten können, so dass eines der hier beschriebenen Verfahren durchgeführt wird. Im Allgemeinen können Ausführungsformen der vorliegenden Offenbarung als Computerprogrammprodukt mit einem Programmcode implementiert werden, wobei der Programmcode zur Ausführung eines der Verfahren dient, wenn das Computerprogrammprodukt auf einem Computer läuft. Der Programmcode kann zum Beispiel auf einem maschinenlesbaren Träger gespeichert sein.

Andere Ausführungsformen umfassen das Computerprogramm zur Durchführung eines der hierin beschriebenen Verfahren, das auf einem maschinenlesbaren Träger gespeichert ist.

Mit anderen Worten, eine Ausführungsform der vorliegenden Offenbarung ist daher ein Computerprogramm mit einem Programmcode zur Durchführung eines der hierin beschriebenen Verfahren, wenn das Computerprogramm auf einem Computer läuft.

Eine weitere Ausführungsform der vorliegenden Offenbarung ist daher ein Speichermedium (oder ein Datenträger oder ein computerlesbares Medium), auf dem das Computerprogramm zur Durchführung eines der hier beschriebenen Verfahren gespeichert ist, wenn es von einem Prozessor ausgeführt wird. Der Datenträger, das digitale Speichermedium oder das aufgezeichnete Medium sind typischerweise greifbar und/oder nicht-übertragbar. Eine weitere Ausführungsform der vorliegenden Offenbarung ist ein Gerät wie hierin beschrieben, das einen Prozessor und das Speichermedium umfasst.

Eine weitere Ausführungsform der vorliegenden Offenbarung ist daher ein Datenstrom oder eine Folge von Signalen, die das Computerprogramm zur Durchführung eines der hier beschriebenen Verfahren darstellen. Der Datenstrom oder die Signalfolge kann beispielsweise so konfiguriert sein, dass er/sie über eine Datenkommunikationsverbindung, z.B. über das Internet, übertragen wird.

Eine weitere Ausführungsform umfasst ein Verarbeitungsmittel, z. B. einen Computer oder ein programmierbares Logikgerät, das so konfiguriert oder angepasst ist, dass es eines der hierin beschriebenen Verfahren durchführen kann.

Eine weitere Ausführungsform umfasst einen Computer, auf dem das Computerprogramm zur Durchführung eines der hierin beschriebenen Verfahren installiert ist.

Eine weitere Ausführungsform gemäß der vorliegenden Offenbarung umfasst eine Vorrichtung oder ein System, die bzw. das so konfiguriert ist, dass sie bzw. es ein Computerprogramm zur Durchführung eines der hierin beschriebenen Verfahren an einen Empfänger überträgt (z. B. auf elektronischem oder optischem Wege). Bei dem Empfänger kann es sich beispielsweise um einen Computer, ein mobiles Gerät, ein Speichergerät oder dergleichen handeln. Die Vorrichtung oder das System kann zum Beispiel einen Dateiserver zur Übertragung des Computerprogramms an den Empfänger umfassen.

In einigen Ausführungsformen kann eine programmierbare Logikvorrichtung (z. B. ein feldprogrammierbares Gate-Array) verwendet werden, um einige oder alle Funktionen der hier beschriebenen Verfahren auszuführen. In einigen Ausführungsformen kann ein feldprogrammierbares Gate-Array mit einem Mikroprozessor zusammenarbeiten, um eines der hier beschriebenen Verfahren durchzuführen. Im Allgemeinen werden die Verfahren vorzugsweise von einem beliebigen Hardware-Gerät durchgeführt.

## Patentansprüche

1. Ein computer-implementiertes Verfahren (100) zum Erzeugen von medizinischen Berichten, insbesondere Arztbriefen, wobei das Verfahren zumindest die folgenden Schritte umfasst:
Empfangen (102) von medizinischen Informationen über einen Patienten, wobei die empfangenen medizinischen Informationen in natürlichsprachlichen Stichpunkten formuliert sind; und
Erzeugen (104) von natürlichsprachlichem Fließtext, welcher die empfangenen medizinischen Informationen widerspiegelt, für einen medizinischen Bericht durch ein trainiertes generatives Sprachmodell.

2. Das Verfahren nach Anspruch 1, wobei das generative Sprachmodell so konfiguriert ist, dass der erzeugte natürlichsprachliche Fließtext alle und nur die empfangenen medizinischen Informationen widerspiegelt.

3. Das Verfahren nach irgendeinem der Ansprüche 1 oder 2, wobei das generative Sprachmodell so konfiguriert ist, dass der natürlichsprachliche Fließtext die empfangenen Informationen deterministisch widerspiegelt.

4. Das Verfahren nach irgendeinem der vorhergehenden Ansprüche,
wobei der Schritt des Empfangens von medizinischen Informationen umfasst:
Empfangen von zumindest einer Benutzereingabe in einer grafischen Benutzeroberfläche, insbesondere durch medizinisches Fachpersonal während einer Visite des Patienten; und/oder
Empfangen von zumindest einem elektronischen Datensatz aus einem Krankenhausinformationssystem, wobei der zumindest eine elektronische Datensatz insbesondere "Health Level 7"-konform und/oder "Fast Healthcare Interoperability Resources"-konform ist; und/oder
wobei das Verfahren ferner den folgenden Schritt umfasst:
Erzeugen des medizinischen Berichts als elektronische Datei, welche den erzeugten natürlichsprachlichen Fließtext enthält.

5. Das Verfahren nach irgendeinem der vorhergehenden Ansprüche, wobei die empfangenen medizinischen Informationen zumindest eine medizinische Information umfassen, welche einer der folgenden Kategorien angehört: Geschlecht, Leitsymptom, Diagnosen, Behandlungen, Medikamente, Befunde und Verlaufsdokumentation.

6. Das Verfahren nach dem vorhergehenden Anspruch 5, welches ferner den folgenden Schritt umfasst:
Kategorisieren der empfangenen medizinischen Informationen in die Kategorien, vorzugsweise mittels eines Klassifikators.

7. Das Verfahren nach irgendeinem der vorhergehenden Ansprüche, welches ferner den folgenden Schritt umfasst:
Erzeugen einer Eingabekodierung für das generative Sprachmodell basierend auf den empfangenen medizinischen Informationen, wobei die Eingabekodierung die medizinischen Informationen so kodiert, dass eine maximale Eingabelänge des generativen Sprachmodells berücksichtigt wird.

8. Das Verfahren nach Anspruch 5 oder 6, jeweils kombiniert mit Anspruch 7, wobei die Eingabekodierung so erzeugt wird, dass jede medizinische Information in der Eingabekodierung einem vorgegebenen Kategorie-Kürzel, welches die jeweilige Kategorie anzeigt, zugeordnet ist;
wobei die Eingabekodierung so erzeugt wird, dass die Eingabekodierung für zumindest eine der Kategorien mehrere medizinische Informationen derselben Kategorie enthält, wobei das Kategorie-Kürzel nur einmal in der Eingabekodierung enthalten ist;
wobei, optional, die Eingabekodierung so erzeugt wird, dass die mehreren medizinischen Informationen derselben Kategorie durch ein vorgegebenes Trenn-Kürzel getrennt sind.

9. Das Verfahren nach irgendeinem der vorhergehenden Ansprüche 7-8, wobei die Eingabekodierung so erzeugt wird, dass zumindest einer medizinischen Information in der Eingabekodierung ein Zeitstempel zugeordnet ist, insbesondere nach der medizinischen Information und durch ein vorgegebenes Trenn-Kürzel getrennt.

10. Das Verfahren nach irgendeinem der vorhergehenden Ansprüche 7-9, wobei das Kategorie-Kürzel und/oder das Trenn-Kürzel genau ein Zeichen enthält.

11. Ein generatives Sprachmodell zum Erzeugen von medizinischen Berichten, insbesondere Arztbriefen, wobei das generative Sprachmodell zum Erzeugen von natürlichsprachlichem Fließtext für einen medizinischen Bericht konfiguriert ist, wobei der erzeugte natürlichsprachliche Fließtext medizinische Informationen über einen Patienten widerspiegelt, wobei die medizinischen Informationen in natürlichsprachlichen Stichpunkten formuliert sind;
wobei, optional, das generative Sprachmodell zur Verwendung im Verfahren (100) nach irgendeinem der vorhergehenden Ansprüche 1-10 konfiguriert ist.

12. Eine Eingabekodierungs-Datenstruktur für das generative Sprachmodell nach Anspruch 11, wobei die Eingabekodierung nach irgendeinem der vorhergehenden Ansprüche 6-10 konfiguriert ist.

13. Ein Verfahren (200) zum Trainieren des generativen Sprachmodells nach Anspruch 11, wobei das Verfahren zumindest die folgenden Schritte umfasst:
Bereitstellen (202) von Trainingsdatensätzen, welche jeweils umfassen:
eine Eingabekodierungs-Datenstruktur nach Anspruch 12;
einen natürlichsprachlichen Fließtext für einen medizinischen Bericht; und
Trainieren (204) des generativen Sprachmodells durch überwachtes Lernen mit den Trainingsdatensätzen.

14. Eine Datenverarbeitungsvorrichtung umfassend Mittel zum Durchführen des Verfahrens nach irgendeinem der Ansprüche 1-10 oder 13.

15. Ein Computerprogramm oder ein computerlesbares Medium, auf dem ein Computerprogramm gespeichert ist, wobei das Computerprogramm Anweisungen umfasst, die, wenn das Programm von einem Computer ausgeführt wird, den Computer veranlassen, das Verfahren nach irgendeinem der Ansprüche 1-10 auszuführen.
